(19) Europäisches Patentamt
European Patent Office
Office européen des brevets

(11) **EP 3 678 495 B1**

(12) **FASCICULE DE BREVET EUROPEEN**

(45) Date de publication et mention
de la délivrance du brevet:
**19.11.2025 Bulletin 2025/47**

(21) Numéro de dépôt: **18773813.3**

(22) Date de dépôt: **05.09.2018**

(51) Classification Internationale des Brevets (IPC):
**A23K 10/20** *(2016.01)* **A23K 40/25** *(2016.01)*
**A23K 50/80** *(2016.01)* **A61P 43/00** *(2006.01)*

(52) Classification Coopérative des Brevets (CPC):
**A23K 10/20; A23K 40/25; A23K 50/80; A61P 43/00**

(86) Numéro de dépôt international:
**PCT/FR2018/052166**

(87) Numéro de publication internationale:
**WO 2019/048776 (14.03.2019 Gazette 2019/11)**

(54) **POUDRE D'INSECTES POUR PREVENIR OU REDUIRE LE STRESS DES POISSONS PENDANT L'ELEVAGE**

AUS INSEKTEN HERGESTELLTES PULVER ZUR VERHINDERUNG ODER VERMINDERUNG VON STRESS IN GEZÜCHTETEN FISCHEN

POWDER MADE OF INSECTS, FOR THE PREVENTION OR REDUCTION OF STRESS IN FISH BEING FARMED

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.09.2017 FR 1758220**

(43) Date de publication de la demande:
**15.07.2020 Bulletin 2020/29**

(73) Titulaire: **Ynsect**
**91058 Evry Cedex (FR)**

(72) Inventeurs:
• **MOTTE, Constant**
**59510 Hem (FR)**
• **ARMENJON, Benjamin**
**75013 Paris (FR)**

(74) Mandataire: **Santarelli**
**Tour Trinity**
**1 bis Place de la Défense**
**92400 Courbevoie (FR)**

(56) Documents cités:
WO-A1-2015/118253    WO-A1-2017/068278
WO-A1-98/34498    WO-A2-2009/140327
CN-A- 105 831 485

• **SANCHEZ-MUROS MA J ET AL: "Nutritional evaluation of Tenebrio molitor meal as fishmeal substitute for tilapia (Oreochromis niloticus) diet", AQUACULTURE NUTRITION, vol. 22, no. 5, October 2016 (2016-10-01), pages 943 - 955, XP009505213, DOI: 10.1111/ANU.12313**
• **MARK D. FINKE ET AL: "Complete nutrient content of four species of commercially available feeder insects fed enhanced diets during growth : Complete Nutrient Content of Four Species of Feeder Insects", ZOO BIOLOGY, vol. 34, no. 6, 1 November 2015 (2015-11-01), US, pages 554 - 564, XP055532095, ISSN: 0733-3188, DOI: 10.1002/zoo.21246**

**Description**

L'invention concerne une poudre d'insectes pour son utilisation pour prévenir ou réduire le stress et la mortalité des poissons pendant leur élevage. WO2009140327 décrit le traitement du stress provenant de la transition de l'eau douce vers l'eau salée des jeunes poissons de la famille Salmonidae, par une nutrition améliorée en acide arachidonique.

Par « poudre d'insectes », on entend une composition, sous forme de particules, préparée uniquement à partir d'insectes et éventuellement d'eau.

Le taux d'humidité résiduel de la poudre d'insectes est compris entre 2 et 15%, de préférence entre 5 et 10%, plus préférentiellement, entre 4 et 8%. Ce taux d'humidité peut par exemple être déterminé selon la méthode issue du règlement CE 152/2009 du 27-01-2009 (103 °C / 4 h).

On notera que dans le cadre de la présente demande, et sauf stipulation contraire, les gammes de valeurs indiquées s'entendent bornes incluses.

Dans toute la demande, lorsqu'aucune date n'est précisée pour un règlement, une norme ou une directive, il s'agit du règlement, de la norme ou de la directive en vigueur à la date de dépôt.

Lorsque la poudre d'insectes est broyée à une taille de particules acceptable pour l'alimentation humaine ou animale, celle-ci peut être désignée sous l'appellation « farine d'insectes ». Par « taille de particules acceptable pour l'alimentation humaine ou animale », on vise une taille de particules comprise entre 100 $\mu$m et 1,5 mm, préférentiellement comprise entre 300 $\mu$m et 1 mm, plus préférentiellement entre 500 et 800 $\mu$m.

Par « insectes », on désigne notamment les coléoptères, les diptères, les lépidoptères, les orthoptères, les hyménoptères, les dictyoptères regroupant notamment les blattoptères, y inclus isoptères, et les mantoptères, les phasmoptères, les hémiptères, les hétéroptères, les éphéméroptères et les mécoptères, ou leurs mélanges, de préférence les coléoptères.

Préférentiellement, les coléoptères appartiennent aux familles des *Tenebrionidae, Melolonthidae, Dermestidae, Coccinellidae, Cerambycidae, Carabidae, Buprestidae, Cetoniidae, Dryophthoridae,* ou leurs mélanges.

Plus préférentiellement, il s'agit des coléoptères suivants : *Tenebrio molitor, Alphitobius diaperinus, Zophobas morio, Tenebrio obscurus, Tribolium castaneum et Rhynchophorus ferrugineus,* ou leurs mélanges, encore plus préférentiellement *Tenebrio molitor.*

La poudre d'insectes visée par l'invention est une poudre de coléoptères du genre *Tenebrio molitor.*

Avantageusement, la poudre d'insectes est obtenue à partir du stade larvaire des espèces d'insectes visées ci-avant.

Par élevage, on vise la production puis l'entretien et la croissance des poissons, depuis le stade alevin jusqu'au stade adulte.

Plus particulièrement, l'élevage visé par l'invention est un élevage à visée commerciale, permettant la production intensive de poissons, tel qu'un élevage en espace clos (bassins, nasses ou cages).

Par stress, on vise plus particulièrement le stress dû à des manipulations (notamment celles désignées sous l'appellation « commercial condition handling ») telles que les manipulations lors de la récupération des poissons dans leur espace clos, lors du transfert d'un espace clos à un autre, lors du transfert jusqu'à l'usine de transformation et d'abattage, et lors de l'abattage lui-même.

L'invention concerne la poudre d'insectes pour son utilisation pour prévenir ou réduire le stress et la mortalité des poissons suite à (ou résultant de) leur transfert de l'eau douce à l'eau de mer ou eau salée pendant l'élevage.

Les poissons visés par l'invention sont les poissons appartenant à la famille *Salmonidae,* qui sont destinés à être transférés, pendant leur élevage, de l'eau douce à l'eau de mer.

Ces poissons sont donc des poissons qui, pendant leur cycle de vie à l'état sauvage, migrent de l'eau douce à l'eau de mer.

Ces poissons se reproduisent dans l'eau douce, et effectuent l'essentiel de leur croissance dans l'eau de mer. Ces poissons sont La poudre d'insectes est utilisée, selon l'invention, pour prévenir ou réduire le stress et la mortalité pendant l'élevage, des poissons appartenant à la famille *Salmonidae.*

De préférence, les poissons appartiennent au genre *Salmo, Salvelinus, Onchorynchus,* et/ou *Hucho,* plus préférentiellement *Salmo.*

Les espèces particulièrement préférées selon l'invention sont : *Salmo salar* (Saumon atlantique), *Salmo trutta* (Truite brune ou Truite commune), *Oncorhynchus kisutch* (Saumon pacifique), *Oncorhynchus tshawytscha* (Saumon royal), *Onchorynchus mykiss* (Truite arc-en-ciel) et *Salvelinus alpinus* (Omble chevalier).

La smoltification désigne l'ensemble des processus physiques et physiologiques (notamment métaboliques) permettant aux poissons provenant d'eau douce de s'adapter aux conditions de vie dans l'eau de mer.

En effet, suite à leur transfert dans l'eau de mer, les poissons peuvent être soumis à des conditions de stress, telles que des conditions de stress dues au changement de milieu (salinité, régime alimentaire, surpeuplement) auxquelles ils doivent s'adapter. L'intensité des conditions de stress pourra varier selon la mise en œuvre de l'élevage. On parlera par exemple de conditions de stress minimales (conditions expérimentales comprenant par exemple des précautions de manipulation) ou, pour des conditions de stress plus intenses, de conditions de stress standards d'élevage.

Cependant, certains poissons ne réussissent pas à s'adapter à ces conditions, et meurent.

Ce défaut d'adaptation ou d'acclimatation des poissons aux conditions de vie dans l'eau de mer ou eau salée peut donc être pallié en utilisant une poudre d'insectes.

L'invention concerne donc également une poudre d'insectes pour son utilisation pour prévenir ou réduire la mortalité des poissons appartenant à la famille *Salmonidae* pendant leur élevage.

Plus particulièrement, l'invention concerne une poudre d'insectes pour son utilisation pour prévenir ou réduire la mortalité ces poissons suite à (ou résultant de) leur transfert de l'eau douce à l'eau de mer ou salée pendant leur élevage.

Avantageusement, l'administration de poudre d'insectes à ces poissons permet de réduire leur mortalité pendant l'élevage de 5% par rapport à la mortalité de poissons auxquels de la poudre d'insectes n'a pas été administrée.

Par « administration », on entend le fait de faire ingérer à des poissons ou de nourrir des poissons.

De préférence, l'administration de poudre d'insectes permet de prévenir totalement la mortalité de ces poissons pendant l'élevage.

Plus particulièrement, l'administration de poudre d'insectes permet de prévenir totalement la mortalité de ces poissons suite à leur transfert de l'eau douce à l'eau de mer pendant l'élevage.

En effet, il a été montré que le taux de mortalité de ces poissons nourris avec une poudre d'insectes est égal à 0% suite à leur transfert de l'eau douce à l'eau salée, tel que par exemple 3 semaines après ce transfert.

Au contraire, le taux de mortalité de poissons qui n'ont pas été nourris avec une poudre d'insectes mais avec une farine de poisson est égal à au moins 5% suite à leur transfert de l'eau douce à l'eau salée, tel que par exemple 3 semaines après ce transfert.

L'effet de la poudre d'insectes sur la mortalité de ces poissons pendant l'élevage est plus amplement détaillé à l'Exemple 3 ci-après.

Avantageusement, la poudre d'insectes selon l'invention comporte au moins 67% en poids de protéines et au moins 0,1% en poids de chitine, les pourcentages étant donnés sur le poids total de poudre d'insectes.

Par « protéines », on vise la quantité de protéines brutes. La quantification des protéines brutes est bien connue de l'homme du métier. A titre d'exemple, on peut citer la méthode

Dumas ou la méthode Kjeldhal. De préférence, la méthode Dumas, correspondant à la norme NF EN ISO 16634-1 (2008) est utilisée.

Des exemples d'une telle poudre sont décrits dans les Exemples 1 et 2 ci-après.

Préférentiellement, la poudre d'insectes comporte 68% en poids de protéines brutes, plus préférentiellement 70% en poids de protéines brutes, plus préférentiellement 71% en poids de protéines brutes, les pourcentages en poids étant donnés sur le poids total de poudre d'insectes.

Selon l'invention, par « chitine », on entend tout type de dérivé chitinique, c'est-à-dire de dérivé de polysaccharides comportant des unités N-acétyl-glucosamine et des unités D-glucosamines, en particulier les copolymères chitine-polypeptides (parfois désignés sous l'appellation « composite chitine-polypeptides »). Ces copolymères peuvent également être associés à des pigments, souvent de type mélanine.

La chitine serait le deuxième polymère le plus synthétisé dans le monde vivant après la cellulose. En effet, la chitine est synthétisée par de nombreuses espèces du monde vivant : elle constitue en partie l'exosquelette des crustacés et des insectes et la paroi latérale qui entoure et protège les champignons. Plus particulièrement, chez les insectes, la chitine constitue ainsi 3 à 60% de leur exosquelette.

La détermination du taux de chitine est effectuée par extraction de celle-ci. Une telle méthode peut être la méthode AOAC 991.43.

Selon un premier mode de réalisation, la poudre d'insectes selon l'invention comporte au moins 67% en poids de protéines et au moins 5% en poids de chitine, les pourcentages étant donnés sur le poids total de poudre d'insectes.

Préférentiellement, cette poudre d'insecte comporte entre 5 et 16% en poids de chitine, plus préférentiellement entre 8 et 14% de chitine, les pourcentages en poids étant donnés sur le poids total de poudre d'insectes.

Avantageusement, cette poudre d'insectes présente une teneur en cendres inférieure ou égale à 4% en poids sur le poids total de poudre d'insectes, et encore plus avantageusement inférieure ou égale à 3,5%.

Les cendres constituent le résidu résultant de la combustion de la composition selon l'invention.

La méthode de détermination de la teneur en cendres est bien connue de l'homme du métier. De préférence, les cendres ont été déterminées selon la méthode relevant du règlement CE 152/2009 du 27-01-2009.

La teneur en matière grasse de cette poudre d'insectes est de préférence comprise entre 5 et 20% en poids sur le poids total de poudre d'insectes, plus préférentiellement entre 9 et 17%.

Les méthodes de détermination de la teneur en matière grasse sont bien connues de l'homme du métier. A titre d'exemple et de manière préférée, la détermination de cette teneur sera effectuée en suivant la méthode du règlement CE 152/2009.

Les termes « matière grasse » et « lipides » sont utilisés indifféremment dans toute la demande.

Avantageusement, les protéines de cette poudre d'insectes présentent une digestibilité supérieure ou égale à 70%, préférentiellement supérieure ou égale à 85% en poids sur le poids total de protéines brutes.

La digestibilité est une digestibilité pepsique mesurée par la méthode décrite dans la directive 72/199/CE.

Plus préférentiellement, la digestibilité est supérieure ou égale à 86%, encore plus préférentiellement, supérieure ou égale à 88% en poids sur le poids total de protéines brutes.

Avantageusement, cette poudre d'insectes selon l'invention comporte entre 35 et 65% en poids de protéines solubles par rapport au poids total de protéines, et au moins 50% des protéines solubles ont une taille inférieure ou égale à 12400g/mol.

Par « poids total de protéines », on entend le poids de protéines brutes présentes dans la poudre d'insectes selon l'invention.

Par « protéines solubles », on entend, parmi les protéines brutes, celles qui sont solubles dans une solution aqueuse dont le pH est compris entre 6 et 8, avantageusement entre 7,2 et 7,6.

De préférence, la solution aqueuse est une solution tampon dont le pH est compris entre 6 et 8, avantageusement entre 7,2 et 7,6. Préférentiellement, la solution tampon est une solution tampon phosphate NaCl, dont le pH est égal 7,4 +/- 0,2.

Avantageusement, cette poudre d'insectes comporte entre 38 et 60% en poids, de préférence entre 43 et 55% en poids de protéines solubles par rapport au poids total de protéines.

De préférence, au moins 60%, préférentiellement au moins 70% des protéines solubles ont une taille inférieure ou égale à 12400g/mol.

Plus particulièrement, les protéines solubles ont une taille comprise entre 6500 et 12400g/mol.

Avantageusement, moins de 10%, de préférence moins de 8%, plus préférentiellement moins de 6% de protéines solubles ont une taille supérieure ou égale à 29000g/mol.

Cette poudre d'insectes peut être préparée par un procédé comportant les étapes suivantes :

i) abattage des insectes,
ii) pressage des insectes pour obtenir un gâteau de presse, et
iii) broyage du gâteau de presse.

L'abattage des insectes peut être effectué par ébouillantage ou blanchiment, comme cela est plus amplement décrit dans l'Exemple 1 ci-après.

De même, les étapes de pressage et de broyage sont plus amplement décrites dans cet Exemple.

Enfin, le procédé de préparation peut comporter en outre une étape de séchage du gâteau de presse.

L'étape de séchage est avantageusement réalisée après l'étape de pressage et avant l'étape de broyage et est également décrite plus amplement dans l'Exemple 1 ci-après.

Selon un second mode de réalisation, la poudre d'insectes selon l'invention comporte au moins 71% en poids de protéines et comporte entre 0,1 et 2% en poids de chitine, les pourcentages étant donnés sur le poids total de poudre d'insectes.

De préférence, cette poudre d'insectes a une teneur en protéines supérieure ou égale à 72% en poids, plus préférentiellement supérieure ou égale à 74% en poids, encore plus préférentiellement supérieure ou égale à 75% en poids, sur le poids sec total de poudre.

Plus particulièrement, cette poudre a une teneur en chitine comprise entre 0,5 et 3% en poids, plus préférentiellement comprise entre 0,8 et 2% en poids, encore plus préférentiellement comprise entre 0,8 et 1,7% en poids sur le poids sec total de poudre.

De préférence, cette poudre comprend entre 5 et 20% en poids, de préférence entre 7 et 17% en poids de lipides, sur le poids sec total de poudre.

Plus particulièrement, cette poudre comprend entre 1 et 10% en poids, de préférence entre 2 et 6% en poids de cendres, sur le poids sec total de poudre.

Avantageusement, les protéines de cette poudre d'insectes présentent une digestibilité supérieure ou égale à 70%, préférentiellement supérieure ou égale à 85% en poids sur le poids total de protéines brutes.

Plus préférentiellement, la digestibilité est supérieure ou égale à 88%, encore plus préférentiellement, supérieure ou égale à 92% en poids sur le poids total de protéines brutes.

Cette poudre d'insectes peut être préparée par un procédé comportant les étapes suivantes :

- abattage des insectes,
- la séparation des cuticules de la partie molle des insectes,
- la séparation de la partie molle des insectes en une fraction solide, une fraction huileuse et une fraction aqueuse,
- le séchage de la fraction solide pour obtenir une fraction solide sèche;
- le broyage de la fraction solide sèche pour obtenir une poudre d'insectes.

L'abattage des insectes peut être effectué par ébouillantage ou blanchiment, comme cela est plus amplement décrit dans l'Exemple 1 ci-après.

La cuticule est la couche externe (ou exosquelette) sécrétée par l'épiderme des insectes. Elle est en général formée de trois couches : l'épicuticule, l'exocuticule et l'endocuticule.

Par « partie molle », on vise la chair (comportant notamment les muscles et les viscères) et le jus (comportant notamment les liquides biologiques, l'eau et l'hémolymphe) des insectes. En particulier, la partie molle ne consiste pas en le jus des insectes.

La séparation des cuticules de la partie molle des insectes peut être effectuée à l'aide d'un filtre presse ou d'un séparateur à bande.

Par séparateur à bande, on vise un dispositif qui comporte une bande de serrage (ou bande presseuse) et un tambour perforé.

La poudre d'insectes selon l'invention est obtenue à partir d'une espèce d'insectes appartenant à l'ordre des coléoptères, de préférence à partir de l'espèce *Tenebrio molitor,* et ce quel que soit le mode de réalisation de l'invention. La poudre d'insecte selon l'invention est alors une poudre de coléoptères, de préférence une poudre de *Tenebrio molitor.*

L'invention vise donc plus particulièrement une poudre de coléoptères, de préférence de *Tenebrio molitor,* pour son utilisation pour réduire le stress des poissons appartenant à la famille *Salmonidae* pendant l'élevage, plus particulièrement pour prévenir ou réduire la mortalité de ces poissons pendant l'élevage, suite à leur transfert de l'eau douce à l'eau de mer.

L'invention concerne par ailleurs l'utilisation d'une poudre d'insectes pour prévenir ou réduire le stress des poissons pendant leur élevage, résultant de leur transfert de l'eau douce à l'eau de mer pendant ledit élevage.

L'invention concerne par ailleurs l'utilisation d'une poudre d'insectes pour prévenir ou réduire la mortalité des poissons appartenant à la famille *Salmonidae* pendant leur élevage.

Plus particulièrement, l'invention concerne l'utilisation d'une poudre d'insectes pour prévenir ou réduire la mortalité de ces poissons suite à leur transfert de l'eau douce à l'eau de mer pendant leur élevage.

Ainsi, la poudre d'insectes est utilisée, selon l'invention, au moins pendant la période d'élevage en eau douce de ces poissons, c'est-à-dire avant le transfert des poissons de l'eau douce à l'eau de mer.

Avantageusement, cette poudre d'insectes est également utilisée pour nourrir les poissons suite à ce transfert, de préférence pendant toute la durée de l'élevage.

La poudre d'insectes présente avantageusement les caractéristiques décrites ci-avant.

Les poissons sont avantageusement les poissons préférés décrits ci-avant.

L'invention vise également un procédé d'élevage de poissons appartenant à la famille *Salmonidae* dans lequel, au cours de l'élevage, les poissons sont transférés d'une eau douce à une eau salée, et dans lequel on administre aux poissons de la poudre d'insectes dans les 10 jours précédant et/ou dans les 10 jours suivant le transfert des poissons de l'eau douce à l'eau salée.

Les poissons visés par le procédé d'élevage selon l'invention sont ceux préférés décrits ci-avant et la poudre d'insectes présente avantageusement les caractéristiques décrites ci-avant et notamment, la poudre d'insectes est une poudre de coléoptères, de préférence une poudre de *Tenebrio molitor.*

De préférence, la poudre d'insectes est administrée dans les 15 jours, plus préférentiellement dans les 25 jours précédant le transfert des poissons.

De préférence, la poudre d'insectes est administrée dans les 15 jours, plus préférentiellement dans les 25 jours suivant le transfert des poissons.

Avantageusement, la poudre d'insectes est administrée dans les jours mentionnés ci-avant précédant et suivant le transfert des poissons.

Avantageusement, la poudre d'insectes est administrée aux poissons de façon journalière, de préférence plusieurs fois par jours.

Plus particulièrement, la poudre d'insectes administré aux poissons constitue au moins 5% en poids, de préférence au moins 10% en poids, préférentiellement au moins 15% en poids, encore plus préférentiellement au moins 20% en poids sur le poids total de leur régime alimentaire.

Dans la présente demande, par « régime alimentaire », on entend l'ensemble des constituants administrés aux poissons, selon des proportions données, les constituants pouvant être administrés concomitamment ou séparément.

L'invention concerne également un régime alimentaire pour poissons appartenant à la famille *Salmonidae* comportant au moins 5% en poids, de préférence au moins 10% en poids, préférentiellement au moins 15% en poids, encore plus préférentiellement au moins 20% en poids de poudre d'insectes définis dans le revendications 1, 2, 9 et 10 sur le poids total de leur régime alimentaire.

La poudre d'insectes selon l'invention peut par exemple être utilisée comme alternative à la farine de poisson généralement administrée dans le régime alimentaire des poissons. Elle peut remplacer partiellement ou en totalité la farine de poisson. Préférentiellement, la poudre d'insectes remplace la farine de poisson à 25% ou plus, en poids de farine de poisson, de préférence à 50% ou plus, en poids de farine de poisson.

La substitution de la farine de poisson par de la poudre d'insectes permet de prévenir ou réduire le stress, et plus particulièrement la mortalité des poissons, pendant l'élevage, suite à leur transfert de l'eau douce à l'eau de mer pendant l'élevage.

Préférentiellement, la poudre d'insectes remplace 50% de la farine de poisson généralement administrée aux poissons.

La poudre d'insectes peut également remplacer la totalité de la farine de poisson généralement administrée aux poissons.

Avantageusement, le régime alimentaire selon l'invention comprend une poudre d'insectes comportant au moins 71% en poids de protéines et comprenant entre 0,1 et 2% en poids de chitine, les pourcentages étant donnés sur le poids total de poudre d'insectes.

Les autres constituants du régime alimentaire sont avantageusement choisis parmi la farine de poisson, la farine de soja, le pois, le blé, le maïs, le gluten de blé, le gluten de maïs, les concentrés de protéines végétales tels que de soja, la lécithine de soja, les huiles (notamment de poisson, colza), les vitamines, les minéraux, les antioxydants, les pigments alimentaires naturels notamment les caroténoïdes tels que l'astaxanthine, les acides aminés tels que la méthionine, la lysine, la thréonine, et/ou les additifs alimentaires tels que les épaississants (gomme de guar), le phosphate monosodique.

Les vitamines et/ou les minéraux peuvent, par exemple, être apportés sous forme de prémix.

L'invention concerne également un procédé visant à prévenir ou réduire le stress et plus particulièrement à prévenir ou réduire la mortalité des poissons appartenant à la famille *Salmonidae* durant l'élevage, comprenant l'administration à ces poissons d'une poudre d'insectes étant une poudre de coléoptères.

Cette poudre d'insectes présente avantageusement les caractéristiques décrites ci-avant.

La poudre d'insectes permet également de favoriser ou augmenter la prise de poids des poissons pendant l'élevage.

Plus particulièrement, la poudre d'insectes permet de favoriser ou augmenter la prise de poids de ces poissons pendant la période d'élevage en eau douce des poissons, c'est-à-dire avant le transfert des poissons de l'eau douce à l'eau de mer.

L'effet de l'utilisation de poudre d'insectes sur la prise de poids de poissons est montré dans l'Exemple 3.

L'invention concerne par ailleurs, l'utilisation d'une poudre d'insectes comportant au moins 67% en poids de protéines et comprenant au moins 0,1% en poids de chitine, les pourcentages étant donnés sur le poids total de poudre d'insectes, comme complément alimentaire dans la nutrition des poissons.

La poudre d'insectes utilisée comme complément alimentaire dans la nutrition des poissons peut comporter au moins 67% en poids de protéines et au moins 5% en poids de chitine, les pourcentages étant donnés sur le poids total de poudre d'insectes. Il s'agit alors de la poudre d'insectes du premier mode de réalisation décrit ci-avant, y inclus toutes les caractéristiques avantageuses, particulières et préférées et son procédé d'obtention.

Alternativement, la poudre d'insectes utilisée comme complément alimentaire dans la nutrition des poissons peut comporter au moins 71% en poids de protéines et comprenant entre 0,1 et 2% en poids de chitine, les pourcentages étant donnés sur le poids total de poudre d'insectes. Il s'agit alors de la poudre d'insectes du second mode de réalisation décrit ci-avant, y inclus toutes les caractéristiques avantageuses, particulières et préférées et son procédé d'obtention.

La poudre d'insectes est une poudre de coléoptères, de préférence une poudre de *Tenebrio molitor.*

D'autres caractéristiques et avantages de l'invention, apparaîtront dans les exemples qui suivent, donnés à titre illustratif.

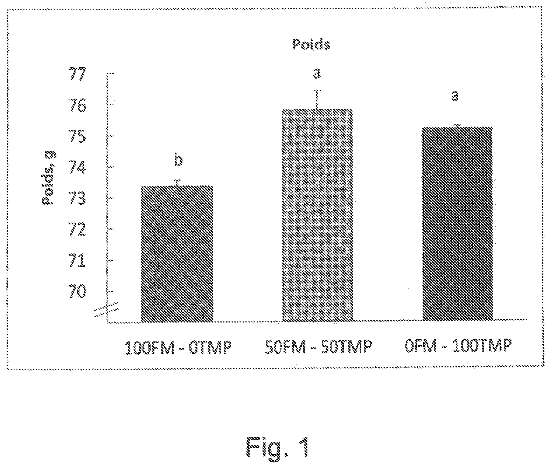

La Figure 1 concerne les poids du groupe « conditions de stress standards » lors du transfert de l'eau douce à l'eau de mer, correspondant à des poissons recevant un régime alimentaire choisi parmi trois régimes différents comprenant de la farine de poisson remplacée par 0% de poudre d'insectes (100FM-0TMP aussi appelé régime alimentaire A), 50% de poudre d'insectes (50FM-50TMP aussi appelé régime alimentaire B) ou 100% de poudre d'insectes (0FM-100TMP aussi appelé régime alimentaire C).

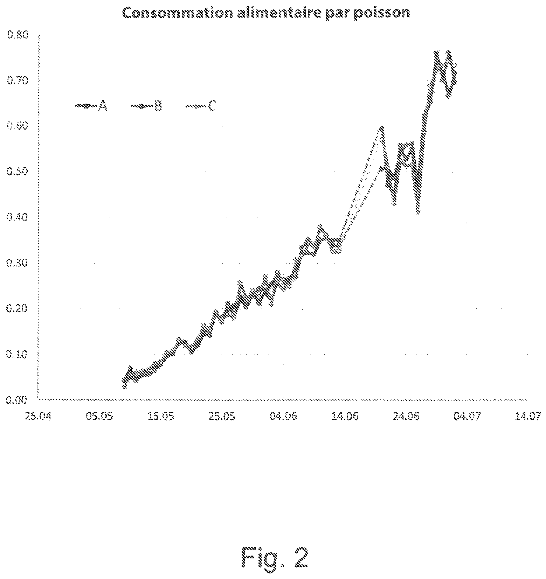

La Figure 2 concerne la consommation alimentaire de poissons nourris avec un régime alimentaire A, B ou C après transfert de l'eau douce à l'eau de mer.

## EXEMPLE 1 : Procédé de préparation d'une poudre d'insectes

La composition selon l'invention est préparée à partir de larves de *Tenebrio molitor.* A réception des larves, ces dernières peuvent être stockées à 4°C pendant 0 à 15 jours dans leurs bacs d'élevages avant l'abattage sans dégradation majeure. Le poids des larves par rapport à l'âge des larves utilisées est variable et par conséquent leur composition peut varier, comme cela est illustré dans le Tableau 1 ci-après :

**Tableau 1** : Composition biochimique des larves de *Tenebrio molitor* selon leur poids.

| Biomasse (Insectes) | mg | 23 | 35 | 58 | 80 | 108 | 154 |
|---|---|---|---|---|---|---|---|
| Matière sèche | %* | 34 | 34 | 34,2 | 37,9 | 39,6 | 39,5 |
| Cendres | %* | 1,59 | 1,52 | 1,6 | 1,75 | 1,67 | 1,43 |
| Protéines brutes | %* | 22,6 | 22,2 | 22 | 23,2 | 23,1 | 23,2 |
| Lipides | %* | 6,62 | 6,88 | 7,98 | 10,3 | 10,9 | 11,7 |
| * Les % sont exprimés en poids sec par rapport au poids humide de larves. | | | | | | | |

### • Etape 1 : Blanchiment des insectes

Les larves vivantes (+ 4°C à + 25°C) sont convoyées en couche d'épaisseur comprise entre 2 et 10 cm, sur un tapis à bande perforé (1mm) jusqu'à une chambre de blanchiment. Les insectes sont ainsi blanchis à la vapeur (buses ou lit de vapeur) à 98°C ou bien à l'eau à 100°C (buses d'aspersion) ou en mode mixte (eau + vapeur). Le temps de séjour dans la chambre de blanchiment est compris entre 1 à 15 minutes, idéalement 5 min.
La température des larves en sortie de blanchiment est comprise entre 75°C et 98°C.

### • Etape 2 : Pressage

Les larves, une fois blanchies, sont convoyées jusqu'à la trémie d'alimentation d'une presse mono-vis continue. Les larves lors du passage en presse sont maintenues à une température supérieure à 70°C pour augmenter les rendements de déshuilage. Le principe de déshuilage est de mettre en pression la matière à l'intérieur d'une cage cylindrique au moyen d'un arrangement de vis et de bagues disposées sur l'axe central. La cage est tapissée à l'intérieur de barreaux répartis en section et maintenus écartés par des espaces d'épaisseurs différentes suivant la zone de travail. Les interstices ainsi ménagés permettent l'écoulement d'une fraction huile et limitent le passage de la matière dite « sèche », la fraction protéique, que l'on appellera « gâteau de presse », participant de la sorte à la mise en pression.
Les rendements de pressage obtenus sont compris entre 48 et 55%.

$$R_{gâteau} = (masse_{gâteau} / masse_{jus} + masse_{gâteau})$$

Le gâteau de presse obtenu contient 35 à 40% de matières sèches, 67 à 75% de protéines et 13 à 17% de matières grasses, les pourcentages en poids étant donnés sur le poids sec de gâteau de presse.

### • Etape 3 : Séchage

Le gâteau de presse est ensuite disposé sur plateau en couche fine (2 cm environ) et, est séché en air ventilé/brassé à 90°C pendant 5 heures afin d'obtenir un gâteau de presse ayant une teneur en matière sèche supérieure à 92%.
Cette étape permet de se prémunir de toute contamination ayant eu lieu depuis l'abattage.
L'Aw (activité en eau) en sortie séchage est de 0,35. Les résultats microbiologiques montrent une absence de Salmonella spp (méthode : IRIS Salmonella BKR 23/07-10/11) et des valeurs en Entérobactéries inférieures à 10 UFC/g (méthode : NF ISO 2128-2, décembre 2004, 30°C et 37°C).

### • Etape 4 : Broyage

Le gâteau de presse séché, comportant majoritairement des protéines, est enfin broyé à l'aide d'un broyeur à marteau continu (6 mobiles réversibles - épaisseur 8 mm). Le broyeur est alimenté par une trémie avec trappe de réglage de débit (180kg/h). La grille perforée utilisée pour contrôler la granulométrie en sortie est de 0,8 mm. La vitesse de rotation du moteur est de 3000tr/min (motorisation électrique, puissance absorbée 4kW (5,5 CV)).

### EXEMPLE 2 : Caractérisation de la poudre d'insectes obtenue dans l'Exemple 1

La poudre d'insectes préparée à l'Exemple 1 a été caractérisée.

### 1. Analyses

1.1 Détermination du taux d'humidité

Le taux d'humidité est déterminé selon la méthode issue du règlement CE 152/2009 du 27-01-2009 (103 °C / 4 h).

1.2 Détermination de la quantité de protéines brutes

Les protéines brutes sont déterminées selon la méthode, dite de Dumas, et correspondant à la norme NF EN ISO 16634-1 (2008).

1.3 Détermination de la quantité de chitine

Les fibres alimentaires de la farine des insectes sont essentiellement composées de chitine, cette dernière a donc été dosée suivant la méthode AOAC 991.43. Les valeurs ainsi obtenues sont par conséquent légèrement surestimées.

1.4 Détermination de la quantité de matière grasse

La matière grasse a été déterminée suivant la méthode du règlement CE 152/2009.

1.5 Détermination de la quantité de cendres

Les cendres brutes ont été déterminées selon la méthode relevant du règlement CE 152/2009 du 27-01-2009.

1.6 Détermination de la quantité de phosphore

Le phosphore est dosé par ICP (« induced coupled plasma ») avec étalonnage interne.

1.7 Détermination de l'énergie

La valeur énergétique est obtenue avec les coefficients du règlement UE 1169/201.

1.8 Détermination des quantités en acides aminés et en acides gras

Cette détermination a été effectuée par chromatographie en phase gazeuse après hydrolyse et dérivatisation des acides aminés et acides gras respectivement.

1.9 Détermination de la digestibilité pepsique

La digestibilité pepsique est mesurée par la méthode décrite dans la directive 72/199/CE.

## 2. <u>Résultats</u>

La poudre d'insectes est détaillée dans le Tableau 2 ci-après.

| Macronutriment | Unité | Composition | Acides gras | Unité | Composition |
|---|---|---|---|---|---|
| Humidité | %* | 5,32 | C12:0 | %* | 0,03 |
| Protéine | %* | 67,09 | C14:0 | %* | 0,22 |
| Chitine | %* | 8,0 | C15:0 | %* | 0,01 |
| Matière grasse | %* | 13,6 | C16:0 | %* | 1,33 |
| Cendre | %* | 3,21 | C16:1 | %* | 0,05 |
| Phosphore total | %* | 0,75 | C16:1n-7 | %* | 0,16 |
| Énergie | MJ/ kg | 23,74 | C17:0 | %* | 0,02 |
| | | | C17:1 | %* | 0,01 |

| Acides aminés | Unité | Composition | | | |
|---|---|---|---|---|---|
| | | | C18:0 | %* | 0,35 |
| Arginine | %* | 2,56 | C18:1n-9 | %* | 3,03 |
| Histidine | %* | 1,39 | C18:1n-7 | %* | 0,04 |
| Isoleucine | %* | 2,11 | C18:2n-6 | %* | 2,96 |
| Leucine | %* | 3,99 | C18:2tn-6 | %* | 0,02 |
| Lysine | %* | 3,32 | C18:3n-3 | %* | 0,14 |
| Thréonine | %* | 1,87 | C20:0 | %* | 0,02 |
| Valine | %* | 2,91 | C20:1n-9 | %* | 0,01 |
| Méthionine | %* | 1,43 | C20:2n-6 | %* | 0,01 |
| Cystéine | %* | 0,63 | C22:0 | %* | 0,01 |
| Phénylalanine | %* | 1,98 | | | |
| Tyrosine | %* | 2,68 | | | |
| Taurine | %* | 0,42 | | | |
| Acide aspartique + asparagine | %* | 4,51 | | | |
| Acide glutamique + glutamine | %* | 6,36 | | | |
| Alanine | %* | 3,83 | | | |
| Glycine | %* | 2,54 | | | |
| Proline | %* | 3,18 | | | |
| Serine | %* | 2,94 | | | |

\* Les pourcentages en poids sont exprimés sur le poids total de composition de poudre.

**Tableau 2 :** Composition de la poudre d'insectes

Par ailleurs, une digestibilité pepsique de 90+/-2% est obtenue.

**EXEMPLE 3 : Effet de la poudre d'insectes obtenue à l'Exemple 1 sur la mortalité et la prise de poids des poissons**

1. Matériel et Méthodes

Poissons : saumons atlantique ayant un poids initial de 60 grammes.

Régimes alimentaires : Les poissons reçoivent un régime alimentaire choisi parmi trois régimes différents comprenant de la farine de poisson remplacée par 0% de poudre d'insectes (régime alimentaire A ou « diet A »), 50% de poudre d'insectes (régime alimentaire B ou « diet B ») ou 100% de poudre d'insectes (régime alimentaire C ou « diet C »).

Ces régimes alimentaires sont détaillés ci-après :

|  | Régime A 0% TMP | Régime B 50% TMP | Régime C 100% TMP |
|---|---|---|---|
| Farine de poissons | 20,00 | 10,00 | 0 |
| Farine d'insectes | 0 | 10,00 | 20,00 |
| Blé | 10,75 | 11,30 | 11,75 |
| Gluten de blé | 14,50 | 13,90 | 13,30 |
| Concentré de protéines de soja | 18,00 | 18,00 | 18,00 |
| Gluten de maïs | 8,00 | 8,00 | 8,00 |
| Lécithine de soja | 1,00 | 1,00 | 1,00 |
| Mélange d'huiles | 20,00 | 19,60 | 19,30 |
| Prémix de minéraux | 0,59 | 0,59 | 0,59 |
| Prémix de vitamines | 2,00 | 2,00 | 2,00 |
| Phosphate monosodique | 2,50 | 2,50 | 2,50 |
| Carop. Pink (10% Astax) | 0,05 | 0,05 | 0,05 |
| DL- Méthionine | 0,60 | 0,75 | 0,90 |
| L- Lysine | 1,20 | 1,40 | 1,60 |
| Thr | 0 | 0,10 | 0,20 |
| Total | 100 | 100 | 100 |
| Composition chimique calculée dans l'alimentation (% dans le régime) | | | |
| Protéines | 45,0 | 45,0 | 45,0 |
| Lipides | 24,0 | 24,0 | 24,0 |
| * TMP = poudre d'insectes, (à savoir « *Tenebrio molitor powder* ») **Farine de poissons: Norse-70 LT (producer: 600031 VEDDE AS), Blue Whiting 54,6 North East Atlantic Ocean Ja Nei, Byproduct NVG Herring 24 Norwegian Sea Ja Ja, Byproduct Herring 13,1 Norwegian Sea Ja Ja, Byproduct whitefish 4,4 Norwegian Sea Ja Nei, Byproduct Mackerel 2,8 North Sea Ja Nei, Byproduct whitefish 1,1 Norwegian Sea *** Carop. Pink (10% Astax) = pigment alimentaire naturel à base d'astaxanthine | | | |

Conditions de l'élevage:

Période d'eau douce du 4 avril au 29 avril (12°C) :

Les poissons sont élevés en eau douce dans trois aquariums en utilisant une lumière continue pour synchroniser la smoltification (norme industrielle).

Les conditions d'élevage sont les suivantes :

| Aquariums | 500 L |
|---|---|
| Sortie d'$O_2$ | 80-99 % de saturation |

(suite)

| | |
|---|---|
| Débit d'eau | 12 L/min |
| Vitesse de l'eau | 8,8 cm/s |
| Photopériode | 24 h |
| Durée des nourrissages | 1 min |
| Temps entre les nourrissages | 20 min |
| Nombre de nourrissages par jour | 72 |
| Nourrisseurs | Convoyeur automatique |
| Température de l'eau | 12°C |

Période d'eau de mer du 29 avril au mois de septembre (température identique à celle de l'environnement)

Pour chaque régime alimentaire :

- 120 poissons sont transférés dans trois aquariums coniques (40 poissons par aquarium) dans des conditions de stress minimales, et
- 120 poissons sont transférés dans trois aquariums coniques (40 poissons par aquarium) selon des conditions de stress standards (par exemple, condition de stress standard dû au surpeuplement)

Le nombre total d'aquariums coniques est donc de 18 et on compte 6 aquariums par régime alimentaire.

2. Résultats

2.1. Poids du groupe « conditions de stress standards » lors du transfert de l'eau douce à l'eau de mer

Voir Figure 1.

*FM : Fish Meal ou farine de poissons
*TMP = Poudre d'insectes

2.2. Poids et mortalité du groupe « conditions de stress standards » 3 semaines après le transfert de l'eau douce à l'eau de mer

| Remplacement de farine de poissons par de la poudre d'insectes | Poids | Mortalité |
|---|---|---|
| Régime A, 0% de poudre d'insectes | 109,4 g | 6,7% |
| Régime B, 50% de poudre d'insectes | 109,2 g | 0% |
| Régime C, 100% de poudre d'insectes | 106,3 g | 0% |

2.3. Consommation alimentaire des poissons après transfert Voir Figure 2.

2.4. Poids 4 mois après le transfert

| | Groupe stress standard | Groupe stress minimal | Total |
|---|---|---|---|
| Nombre de poissons | 39-45 | 10 | 49-55 |
| Régime A (contrôle) | 283,3 g ab | 308,1 g a | 287,7 g b |
| Régime B | 289,9 g a | 351,4 g a | 301,1 g a |

(suite)

|  | Groupe stress standard | Groupe stress minimal | Total |
|---|---|---|---|
| Régime C | 271,5 g b | 331,2 g a | 282,4 g b |

**Revendications**

1. Poudre de coléoptères pour son utilisation pour prévenir ou réduire le stress de poissons appartenant à la famille *Salmonidae* pendant leur élevage, ledit stress des poissons résultant de leur transfert de l'eau douce à l'eau de mer pendant ledit élevage.

2. Poudre de coléoptères pour son utilisation pour prévenir ou réduire la mortalité de poissons appartenant à la famille *Salmonidae* pendant leur élevage, ladite mortalité des poissons résultant de leur transfert de l'eau douce à l'eau de mer pendant ledit élevage.

3. Poudre de coléoptères pour son utilisation selon la revendication 1 ou 2, dans laquelle les poissons appartenant à la famille *Salmonidae* sont du genre *Salmo, Salvelinus, Onchorynchus, et/ou Hucho.*

4. Poudre de coléoptères pour son utilisation selon la revendication 3, dans laquelle les poissons sont choisis parmi les espèces suivantes : *Salmo salar, Salmo trutta, Oncorhynchus kisutch, Oncorhynchus tshawytscha, Onchorynchus mykiss* et *Salvelinus alpinus.*

5. Poudre de coléoptères pour son utilisation selon l'une quelconque des revendications 1 à 4, comportant au moins 67% en poids de protéines et au moins 0,1% en poids de chitine, les pourcentages étant donnés sur le poids total de poudre d'insectes.

6. Poudre de coléoptères pour son utilisation selon la revendication 5, comportant au moins 67% en poids de protéines et au moins 5% en poids de chitine, les pourcentages étant donnés sur le poids total de poudre d'insectes.

7. Poudre de coléoptères pour son utilisation selon la revendication 5, comportant au moins 71% en poids de protéines et comprenant entre 0,1 et 2% en poids de chitine, les pourcentages étant donnés sur le poids total de poudre d'insectes.

8. Poudre de coléoptères pour son utilisation selon l'une quelconque des revendications 1 à 7, obtenue à partir de l'espèce *Tenebrio Molitor.*

9. Poudre de coléoptères pour son utilisation dans un procédé d'élevage de poissons appartenant à la famille *Salmonidae* permettant de prévenir ou réduire le stress desdits poissons dans lequel, au cours de l'élevage, les poissons sont transférés d'une eau douce à une eau salée, et dans lequel on administre aux poissons de la poudre de coléoptères dans les 10 jours précédant et/ou dans les 10 jours suivant le transfert des poissons de l'eau douce à l'eau salée.

10. Poudre de coléoptères pour son utilisation dans un procédé d'élevage de poissons appartenant à la famille *Salmonidae* permettant de prévenir ou réduire la mortalité desdits poissons dans lequel, au cours de l'élevage, les poissons sont transférés d'une eau douce à une eau salée, et dans lequel on administre aux poissons de la poudre de coléoptères dans les 10 jours précédant et/ou dans les 10 jours suivant le transfert des poissons de l'eau douce à l'eau salée.

**Patentansprüche**

1. Käferpulver zur Verwendung zur Verhinderung oder Verringerung des Stresses von Fischen, die der Familie Salmonidae angehören, während ihrer Zucht, wobei sich der Stress der Fische aus der Übertragung von Süßwasser in Meerwasser während der Zucht ergibt.

2. Käferpulver zur Verwendung zur Verhinderung oder Verringerung der Sterblichkeit von Fischen, die der Familie Salmonidae angehören, während ihrer Zucht, wobei sich die Sterblichkeit der Fische aus der Übertragung von Süßwasser in Meerwasser während der Zucht ergibt.

3.  Käferpulver zur Verwendung nach Anspruch 1 oder 2, wobei die Fische der Familie Salmonidae der Gattung Salmo, Salvelinus, Onchorynchus und/oder Hucho sind.

4.  Käferpulver zur Verwendung nach Anspruch 3, wobei die Fische aus den folgenden Arten ausgewählt sind: Salmo salar, Salmo trutta, Oncorhynchus kisutch, Oncorhynchus tshawytscha, Onchorynchus mykiss und Salvelinus alpinus.

5.  Käferpulver zur Verwendung nach einem der Ansprüche 1 bis 4, das mindestens 67 Gew.-% Protein und mindestens 0,1 Gew.-% Chitin aufweist, wobei die Prozentsätze auf das Gesamtgewicht des Insektenpulvers angegeben sind.

6.  Käferpulver zur Verwendung nach Anspruch 5, das mindestens 67 Gew.-% Protein und mindestens 5 Gew.-% Chitin aufweist, wobei die Prozentsätze auf das Gesamtgewicht des Insektenpulvers angegeben sind.

7.  Käferpulver zur Verwendung nach Anspruch 5, das mindestens 71 Gew.-% Protein und zwischen 0,1 und 2 Gew.-% Chitin umfasst, wobei die Prozentsätze auf das Gesamtgewicht des Insektenpulvers angegeben sind.

8.  Käferpulver zur Verwendung nach einem der Ansprüche 1 bis 7, gewonnen aus der Art Tenebrio Molitor.

9.  Käferpulver zur Verwendung in einem Zuchtverfahren für Fische, die der Familie Salmonidae angehören, zur Verhinderung oder Verringerung des Stresses der Fische, bei dem die Fische während der Zucht von Süßwasser in Salzwasser übertragen werden und bei dem den Fischen innerhalb von 10 Tagen vor und/oder innerhalb von 10 Tagen nach der Übertragung der Fische von Süßwasser in Salzwasser Käferpulver verabreicht wird.

10. Käferpulver zur Verwendung in einem Zuchtverfahren für Fische, die der Familie Salmonidae angehören, zur Verhinderung oder Verringerung der Sterblichkeit der Fische, bei dem die Fische während der Zucht von Süßwasser in Salzwasser übertragen werden und bei dem den Fischen innerhalb von 10 Tagen vor und/oder innerhalb von 10 Tagen nach der Übertragung der Fische von Süßwasser in Salzwasser Käferpulver verabreicht wird.

**Claims**

1.  Beetle powder for use thereof for the prevention or reduction of stress in fish belonging to the family *Salmonidae* during their rearing, said stress of the fish resulting from their transfer from fresh water to seawater during said rearing.

2.  Beetle powder for use thereof for the prevention or reduction of mortality in fish belonging to the family *Salmonidae* during their rearing, said mortality of the fish resulting from their transfer from fresh water to seawater during said rearing.

3.  Beetle powder for use thereof according to claim 1 or 2, in which the fish belonging to the family *Salmonidae* are from the genus *Salmo, Salvelinus, Onchorynchus,* and/or *Hucho.*

4.  Beetle powder for use thereof according to claim 3, in which the fish are selected from the following species: *Salmo salar, Salmo trutta, Oncorhynchus kisutch, Oncorhynchus tshawytscha, Onchorynchus mykiss* and *Salvelinus alpinus.*

5.  Beetle powder for use thereof according to any one of claims 1 to 4, comprising at least 67% by weight proteins and at least 0.1% by weight chitin, the percentages being given with respect to the total weight of insect powder.

6.  Beetle powder for use thereof according to claim 5, comprising at least 67% by weight proteins and at least 5% by weight chitin, the percentages being given with respect to the total weight of insect powder.

7.  Beetle powder for use thereof according to claim 5, comprising at least 71% by weight proteins and comprising between 0.1 and 2% by weight chitin, the percentages being given with respect to the total weight of insect powder.

8.  Beetle powder for use thereof according to any one of claims 1 to 7, obtained from the species *Tenebrio molitor.*

9. Beetle powder for use in a method for rearing fish belonging to the family *Salmonidae,* for the prevention or reduction of stress in said fish, wherein during their rearing, the fish are transferred from fresh water to salt water, and wherein the beetle powder is administered to the fish in the 10 days preceding and/or in the 10 days following the transfer of the fish from fresh water to salt water.

10. Beetle powder for use in a method for rearing fish belonging to the family *Salmonidae,* for the prevention or reduction of mortality of said fish, wherein during their rearing, the fish are transferred from fresh water to salt water, and wherein the beetle powder is administered to the fish in the 10 days preceding and/or in the 10 days following the transfer of the fish from fresh water to salt water.

Fig. 1

Fig. 2

**EP 3 678 495 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009140327 A